# EUROPEAN PATENT APPLICATION

(11) **EP 4 116 401 A1**
(43) Date of publication of application: **11.01.2023**
(21) Application number: 21765324.5
(22) Date of filing: 03.03.2021
(51) Int. Cl.: C12M 1/26, C12N 5/078, C12N 1/04, C12Q 1/68

(54) **STORAGE CONTAINER FOR CELL-CONTAINING SOLUTION AND STORAGE SOLUTION**

(30) Priority: 05.03.2020 JP 2020038157
(71) Applicant: Sekisui Medical Co., Ltd., Tokyo 103-0027 (JP); Tokuyama Sekisui Co., Ltd., Osaka-Shi, Osaka 530-8565 (JP)
(72) Inventor: GOTOU, Yuuki, Shunan-shi, Yamaguchi 746-0006 (JP); ISOGAWA, Hironobu, Shunan-shi, Yamaguchi 746-0006 (JP); UCHIYAMA, Takaya, Shunan-shi, Yamaguchi 746-0006 (JP); INOUE, Tomonori, Shunan-shi, Yamaguchi 746-0006 (JP)
(74) Representative: Ter Meer Steinmeister & Partner
(86) International application number: PCT/JP2021/008128
(87) International publication number: WO 2021/177344

(57) **Abstract**

Provided is a storage container for cell-containing solutions, which makes it possible to enhance the storage stability of cfDNA contained in a cell-containing solution even when the cell-containing solution is stored under a room temperature environment. The storage container for cell-containing solutions according to the present invention is intended to be used for storing a predetermined amount of a cell-containing solution, the storage container being provided with a container main body and a preservative solution contained in the container main body, in which the storage container has a first configuration such that the preservative solution contains a cell-membrane-permeable compound that has a molecular weight of 100 or less and cannot be frozen at 0°C and, when a mixed solution X is prepared by collecting the predetermined amount of the cell-containing solution in the storage container for cell-containing solutions and mixing the cell-containing solution with the preservative solution, the content of the cell-membrane-permeable compound in the mixed solution X is 1 vol% to 5vol% inclusive, or the storage container has a second configuration such that the preservative solution contains a cell-membrane-impermeable compound having a molecular weight of 300 or more and, when a mixed solution X is prepared by collecting the predetermined amount of the cell-containing solution in the storage container for cell-containing solutions and mixing the cell-containing solution with the preservative solution, the content of the cell-membrane-impermeable compound in the mixed solution X is 0.5 µmol/L to 5 µmol/L inclusive.

## Description

### TECHNICAL FIELD

The present invention relates to a storage container for storing a cell-containing solution. The present invention also relates to a preservative solution which is used for the storage of a cell-containing solution.

### BACKGROUND ART

In a body fluid such as blood, plasma, serum and urine, cell-free DNA (cfDNA) is contained. It is known that cfDNA is present at a higher level in a body fluid from a person having a malignant tumor or a person suffering from an infectious disease compared with that from a normal person (Non-Patent Document 1).

In recent years, tests using cfDNA as a specimen and the like have been performed in the fields of cancer and genetic diseases. A test using cfDNA as a specimen has less burden on patients compared with a test in which an affected tissue is collected from a patient.

In maternal blood, free DNA derived from a fetus (cell free fetal DNA, cffDNA) is contained (Non-Patent Document 2). In prenatal genetic tests, an examination using cffDNA as a specimen has been performed.

A test using cfDNA as a specimen cannot be performed easily unless performed in a specialized facility equipped with a specially designed device such as a qPCR device and a next-generation sequencer (NGS). A cell-containing solution, e.g., blood, collected in a hospital or the like needs to be transported to a specialized facility. Therefore, a several number of days is required from the collection of a cell-containing solution till the analysis of the cell-containing solution. During this procedure, the cell-containing solution is stored in a particular container.

In Patent Documents 1 and 2, a method for collecting cfDNA stably by stabilizing cells, particularly leukocytes, occurring in the whole blood is disclosed.

In Patent Document 3, a vitrification-cryopreservation solution for animal cells is disclosed, which does not contain serum and contains, as a cryoprotectant substance, at least one water-soluble cellulose-based cryoprotectant substance selected from the group consisting of hydroxypropyl cellulose, hydroxypropyl methyl cellulose and hydroxyethyl methyl cellulose. In Patent Document 3, it is described that it is preferred for the vitrification-cryopreservation solution to contain a specific cell-membrane-permeable cryoprotectant substance or a specific cell-membrane-impermeable cryoprotectant substance.

### Related Art Documents

### Patent Documents

Patent Document 1: WO2013/123030A1
Patent Document 2: CN107083382A
Patent Document 3: JP 2011-111406 A

### Non-Patent Documents

Non-Patent Document 1: Karen-Lise Garm Spindler, Ane L.Appelt, Niels Pallisgaard, et al. Cell-free DNA in healthy individuals, noncancerous disease and strong prognostic value in colorectal cancer. Int. J. Cancer: 135, 2984-2991 (2014)
Non-Patent Document 2: Alberry M, Maddocks D, Jones M, et al. Free fetal DNA in maternal plasma in anembryonic pregnancies: confirmation that the origin is the trophoblast. Prenat Diagn. 2007 May; 27(5): 415-8

### SUMMARY OF THE INVENTION

### PROBLEMS TO BE SOLVED BY THE INVENTION

A test using cfDNA contained in a cell-containing solution, e.g., blood, as a specimen has been performed. A cell-containing solution such as blood is collected in a storage container having a preservative solution contained therein, and is then transported to a specialized facility equipped with a qPCR device or a next generation sequencer. The transportation to the specialized facility is sometimes performed under a frozen environment. From the viewpoint of reducing transportation cost and the viewpoint of avoiding the occurrence of troubles during transportation, it is desirable to perform the transportation to a specialized facility under a room temperature environment (e.g., 15°C to 25°C) .

However, in a conventional storage container having a preservative solution contained therein, when a cell-containing solution is stored in the storage container under a room temperature environment, the storage stability of cfDNA contained in the cell-containing solution may be deteriorated. For example, in the conventional storage container, cfDNA may be decomposed by a deoxyribonuclease contained in the cell-containing solution during the storage of the cell-containing solution under a room temperature environment. Furthermore, in the conventional storage container, cells contained in a cell-containing solution may be disrupted or killed to release genomic DNA (gDNA) in the cells into the cell-containing solution during the storage of the cell-containing solution under a room temperature environment. In a conventional storage container having EDTA contained therein, although EDTA inhibits the activation of a DNase to prevent the decomposition of cfDNA to a certain extent, the death or the like of cells cannot be prevented.

In the method disclosed in Patent Document 1, the storage stability of cfDNA contained in a cell-containing solution can be enhanced to a certain extent. However, the present inventors have found that, even in the method disclosed in Patent Document 1, the storage stability of cfDNA is deteriorated, because, for example, the crosslinking between nucleic acid molecules or the crosslinking between a nucleic acid and a protein is caused by formaldehyde released from a formaldehyde donor compound and, as a result, the apparent fragment length of cfDNA is increased. Also, in a prenatal genetic test, it is necessary to distinguish between cfDNA from a fetus (cffDNA) and cfDNA from a mother body. The cffDNA is degraded during the passing of the cffDNA through a maternal tissue such as placenta and, as a result, the fragment length of the cffDNA is decreased compared with cfDNA from a mother body. In a prenatal genetic test, cffDNA and cfDNA are distinguished from each other by utilizing the difference in fragment length therebetween. Therefore, if the crosslinking between the cffDNA and the cfDNA by the action of formaldehyde or the like occurs and, as a result, the fragment length of the cffDNA or the fragment length of the cfDNA is changed, the test results may be significantly affected.

In the method disclosed in Patent Document 2, it is also difficult to sufficiently enhance the storage stability of cfDNA.

In the case where a cell-containing solution such as a body fluid is stored using a conventional vitrification-cryopreservation solution as disclosed in Patent document 3, the vitrification-cryopreservation solution is diluted excessively with the cell-containing solution. Therefore, the effect of the vitrification-cryopreservation solution cannot be achieved satisfactorily. As a result, it becomes difficult to enhance the storage stability of cfDNA contained in the cell-containing solution.

As mentioned above, in the conventional methods, when a cell-containing solution is stored under a room temperature environment, it has been difficult to enhance the storage stability of cfDNA satisfactorily.

An object of the present invention is to provide a storage container for cell-containing solutions, which makes it possible to enhance the storage stability of cfDNA contained in a cell-containing solution even when the cell-containing solution is stored under a room temperature environment. Another object of the present invention is to provide a preservative solution which makes it possible to enhance the storage stability of cfDNA contained in a cell-containing solution even when the cell-containing solution is stored under a room temperature environment.

### MEANS FOR SOLVING THE PROBLEMS

According to a broad aspect of the present invention, a storage container for cell-containing solutions is provided, which is used for storing a predetermined amount of a cell-containing solution, the storage container being provided with a container main body and a preservative solution contained in the container main body, in which the storage container has a first configuration such that the preservative solution contains a cell-membrane-permeable compound that has a molecular weight of 100 or less and cannot be frozen at 0°C and, when a mixed solution X is prepared by collecting the predetermined amount of the cell-containing solution in the storage container for cell-containing solutions and mixing the cell-containing solution with the preservative solution, the content of the cell-membrane-permeable compound in the mixed solution X is 1 vol% or more and 5 vol% or less, or the storage container has a second configuration such that the preservative solution contains a cell-membrane-impermeable compound having a molecular weight of 300 or more and, when a mixed solution X is prepared by collecting the predetermined amount of the cell-containing solution in the storage container for cell-containing solutions and mixing the cell-containing solution with the preservative solution, the content of the cell-membrane-impermeable compound in the mixed solution X is 0.5 µmol/L or more and 5 µmol/L or less.

According to a specific aspect of the storage container for cell-containing solutions of the present invention, the storage container for cell-containing solutions has the first configuration, and the cell-membrane-permeable compound is a polyhydric alcohol.

According to a specific aspect of the storage container for cell-containing solutions of the present invention, the storage container for cell-containing solutions has the first configuration, and the cell-membrane-permeable compound is ethylene glycol, propylene glycol, glycerin, dimethyl sulfoxide, acetamide, 1,3-propanediol or butylene glycol.

According to a specific aspect of the storage container for cell-containing solutions of the present invention, the storage container for cell-containing solutions has the second configuration, and the cell-membrane-impermeable compound is a compound having a molecular weight of 1000 or more.

According to a specific aspect of the storage container for cell-containing solutions of the present invention, the storage container for cell-containing solutions has the second configuration, and the cell-membrane-impermeable compound is polyvinylpyrrolidone, polyethylene glycol, polyvinyl alcohol, a polysaccharide, a derivative of a polysaccharide, a sugar alcohol or Ficoll.

According to a specific aspect of the storage container for cell-containing solutions of the present invention, the storage container for cell-containing solutions has the first configuration, and the preservative solution contains a formaldehyde donor compound as a compound that is different from either of the cell-membrane-permeable compound and the cell-membrane-impermeable compound.

According to a specific aspect of the storage container for cell-containing solutions of the present invention, the preservative solution contains a formaldehyde donor compound as a compound that is different from either of the cell-membrane-permeable compound and the cell-membrane-impermeable compound and, when a solution Y' is prepared by mixing 7.4 g of sodium hypochlorite with 1 L of physiological saline and a mixed solution Y is prepared by collecting the solution Y' in an amount equal to the predetermined amount of the cell-containing solution to be stored in the storage container for cell-containing solutions in the storage container for cell-containing solutions and mixing the solution Y' with the preservative solution, the content of formaldehyde in the mixed solution Y is 100 mg/L or more and 400 mg/L or less.

According to a specific aspect of the storage container for cell-containing solutions of the present invention, the formaldehyde donor compound is DMDM hydantoin or 1-hydroxymethyl-5,5-dimethylhydantoin.

According to a specific aspect of the storage container for cell-containing solutions of the present invention, when a mixed solution Z is prepared by collecting physiological saline in an amount equal to the predetermined amount of a cell-containing solution to be stored in the storage container for cell-containing solutions in the storage container for cell-containing solutions and mixing the physiological saline with the preservative solution, the osmotic pressure in the mixed solution Z is 300 mOsm/L or more and 1100 mOsm/L or less.

According to a specific aspect of the storage container for cell-containing solutions of the present invention, the preservative solution contains an osmotic pressure-controlling agent as a compound that is different from either of the cell-membrane-permeable compound and the cell-membrane-impermeable compound, and the osmotic pressure-controlling agent is glucose or sodium chloride.

According to a specific aspect of the storage container for cell-containing solutions of the present invention, the preservative solution contains a compound having a chelating activity as a compound that is different from either of the cell-membrane-permeable compound and the cell-membrane-impermeable compound, the compound having a chelating activity comprises EDTA, and the content of the EDTA in the mixed solution X is 4 mmol/L or more and 7 mmol/L or less.

According to a specific aspect of the storage container for cell-containing solutions of the present invention, the preservative solution contains a buffering agent as a compound that is different from either of the cell-membrane-permeable compound and the cell-membrane-impermeable compound, the buffering agent comprises glycine, and the content of the glycine in the mixed solution X is 0.5 w/v% or less.

According to a specific aspect of the storage container for cell-containing solutions of the present invention, the storage container for cell-containing solutions has the first configuration and the second configuration.

According to a specific aspect of the storage container for cell-containing solutions of the present invention, the cell-containing solution is blood.

According to a broad aspect of the present invention, a preservative solution for use in the storage of a cell-containing solution is provided, the preservative solution having a third configuration such that a cell-membrane-permeable compound that has a molecular weight of 100 or less and cannot be frozen at 0°C is contained, in which the content of the cell-membrane-permeable compound is 2 vol% or more and 50 vol% or less, or the preservative solution having a fourth configuration such that a cell-membrane-impermeable compound that has a molecular weight of 300 or more is contained, in which the content of the cell-membrane-impermeable compound is 1.0 µmol/L or more and 100 µmol/L or less.

According to a specific aspect of the preservative solution of the present invention, the preservative solution has the third configuration, and the cell-membrane-permeable compound is a polyhydric alcohol.

According to a specific aspect of the preservative solution of the present invention, the preservative solution has the third configuration, and the cell-membrane-permeable compound is ethylene glycol, propylene glycol, glycerin, dimethyl sulfoxide, acetamide, 1,3-propanediol or butylene glycol.

According to a specific aspect of the preservative solution of the present invention, the preservative solution has the fourth configuration, and the cell-membrane-impermeable compound is a compound having a molecular weight of 1000 or more.

According to a specific aspect of the preservative solution of the present invention, the preservative solution has the fourth configuration, and the cell-membrane-impermeable compound is polyvinylpyrrolidone, polyethylene glycol, polyvinyl alcohol, a polysaccharide, a derivative of a polysaccharide, a sugar alcohol or Ficoll.

According to a specific aspect of the preservative solution of the present invention, the preservative solution contains a formaldehyde donor compound as a compound that is different from either of the cell-membrane-permeable compound and the cell-membrane-impermeable compound.

According to a specific aspect of the preservative solution of the present invention, the formaldehyde donor compound is DMDM hydantoin or 1-hydroxymethyl-5,5-dimethylhydantoin.

According to a specific aspect of the preservative solution of the present invention, the preservative solution contains an osmotic pressure-controlling agent as a compound that is different from either of the cell-membrane-permeable compound and the cell-membrane-impermeable compound, and the osmotic pressure-controlling agent is glucose or sodium chloride.

According to a specific aspect of the preservative solution of the present invention, the preservative solution contains a compound having a chelating activity as a compound that is different from either of the cell-membrane-permeable compound and the cell-membrane-impermeable compound, and the compound having a chelating activity comprises EDTA.

According to a specific aspect of the preservative solution of the present invention, the preservative solution contains a buffering agent as a compound that is different from either of the cell-membrane-permeable compound and the cell-membrane-impermeable compound, and the buffering agent comprises glycine.

According to a specific aspect of the preservative solution of the present invention, the preservative solution has the third configuration and the fourth configuration.

According to a specific aspect of the preservative solution of the present invention, the cell-containing solution is blood.

### EFFECT OF THE INVENTION

The storage container for cell-containing solutions according to the present invention is a container which is intended to be used for the storage of a predetermined amount of a cell-containing solution, and is provided with a container main body and a preservative solution that is contained in the container main body. The storage container for cell-containing solutions according to the present invention has a first configuration or a second configuration as mentioned below. The first configuration: the preservative solution contains a cell-membrane-permeable compound that has a molecular weight of 100 or less and cannot be frozen at 0°C, and, when a mixed solution X is prepared by collecting the predetermined amount of the cell-containing solution in the storage container for cell-containing solutions and mixing the cell-containing solution with the preservative solution, the content of the cell-membrane-permeable compound in the mixed solution X is 1 vol% or more and 5 vol% or less. The second configuration: the preservative solution contains a cell-membrane-impermeable compound that has a molecular weight of 300 or more, and, when a mixed solution X is prepared by collecting the predetermined amount of the cell-containing solution in the storage container for cell-containing solutions and mixing the cell-containing solution with the preservative solution, the content of the cell-membrane-impermeable compound in the mixed solution X is 0.5 µmol/L or more and 5 µmol/L or less. In the storage container for cell-containing solutions according to the present invention, because the storage container has the above-mentioned configuration, the storage stability of cfDNA contained in a cell-containing solution can be enhanced even when the cell-containing solution is stored under a room temperature environment.

The preservative solution according to the present invention is one which is intended to be used for the storage of a cell-containing solution. The preservative solution according to the present invention has a third configuration or a fourth configuration as mentioned below. The third configuration: a cell-membrane-permeable compound that has a molecular weight of 100 or less and cannot be frozen at 0°C is contained, and the content of the cell-membrane-permeable compound is 2 vol% or more and 50 vol% or less. The fourth configuration: a cell-membrane-impermeable compound having a molecular weight of 300 or more is contained, and the content of the cell-membrane-impermeable compound is 1.0 µmol/L or more and 100 µmol/L or less. In the preservative solution according to the present invention, because the preservative solution has the above-mentioned configuration, the storage stability of cfDNA contained in a cell-containing solution can be enhanced even when the cell-containing solution is stored under a room temperature environment.

### BRIEF DESCRIPTION OF DRAWINGS

[Fig. 1] Figs. 1(a) to 1(c) show the results of the image analysis of electrophoretic images obtained in the evaluation of storage stability of cfDNA in Example 1.
[Fig. 2] Figs. 2(a) and 2(b) show the results of the image analysis of electrophoretic images obtained in the evaluation of storage stability of cfDNA in Example 2.
[Fig. 3] Figs. 3(a) to 3(c) show the results of the image analysis of electrophoretic images obtained in the evaluation of storage stability of cfDNA in Comparative Example 1; and Fig. 3(d) shows the results of the image analysis of an electrophoretic image obtained in the evaluation of storage stability of cfDNA in Comparative Example 4.
[Fig. 4] Figs. 4(a) and 4(b) show the results of the image analysis of electrophoretic images obtained in the evaluation of storage stability of cfDNA in Comparative Example 2.
[Fig. 5] Figs. 5(a) to 5(c) show the results of the image analysis of electrophoretic images obtained in the evaluation of storage stability of cfDNA in Comparative Example 3.
[Fig. 6] Figs. 6(a) to 6(c) show the results of the image analysis of electrophoretic images obtained in the evaluation of storage stability of cfDNA in Examples 3 to 5, respectively.
[Fig. 7] Figs. 7(a) to 7(c) show the results of the image analysis of electrophoretic images obtained in the evaluation of storage stability of cfDNA in Examples 6 to 8, respectively.
[Fig. 8] Figs. 8(a) to 8(d) show the results of the image analysis of electrophoretic images obtained in the evaluation of storage stability of cfDNA in Examples 9 to 12, respectively.
[Fig. 9] Figs. 9(a) and 9(b) show the results of the image analysis of electrophoretic images obtained in the evaluation of storage stability of cfDNA in Example 13.
[Fig. 10] Figs. 10(a) and 10(b) show the results of the image analysis of electrophoretic images obtained in the evaluation of storage stability of cfDNA in Example 14.
[Fig. 11] Figs. 11(a) and 11(b) show the results of the image analysis of electrophoretic images obtained in the evaluation of storage stability of cfDNA in Example 15.

### MODES FOR CARRYING OUT THE INVENTION

Hereinbelow, the present invention will be described in detail.

The storage container for cell-containing solutions according to the present invention (which is sometimes abbreviated as "storage container", hereinafter) is a container which is intended to be used for the storage of a predetermined amount of a cell-containing solution, and includes a container main body and a preservative solution that is contained in the container main body. The storage container for cell-containing solutions according to the present invention has a first configuration or a second configuration as mentioned below.

The first configuration: The preservative solution contains a cell-membrane-permeable compound that has a molecular weight of 100 or less and cannot be frozen at 0°C, and, when a mixed solution X is prepared by collecting the predetermined amount of the cell-containing solution in the storage container for cell-containing solutions and mixing the cell-containing solution with the preservative solution, the content of the cell-membrane-permeable compound in the mixed solution X is 1 vol% or more and 5 vol% or less.

The second configuration: The preservative solution contains a cell-membrane-impermeable compound that has a molecular weight of 300 or more, and, when a mixed solution X is prepared by collecting the predetermined amount of the cell-containing solution in the storage container for cell-containing solutions and mixing the cell-containing solution with the preservative solution, the content of the cell-membrane-impermeable compound in the mixed solution X is 0.5 µmol/L or more and 5 µmol/L or less.

The preservative solution according to the present invention is one which is intended to be used for the storage of a cell-containing solution. The preservative solution according to the present invention has a third configuration or a fourth configuration as mentioned below.

The third configuration: A cell-membrane-permeable compound that has a molecular weight of 100 or less and cannot be frozen at 0°C is contained, and the content of the cell-membrane-permeable compound is 2 vol% or more and 50 vol% or less.

The fourth configuration: A cell-membrane-impermeable compound having a molecular weight of 300 or more is contained, and the content of the cell-membrane-impermeable compound is 1.0 µmol/L or more and 100 µmol/L or less.

In the storage container and the preservative solution according to the present invention, the cell-containing solution is stored in a state such that the cell-containing solution is mixed with the preservative solution. In the storage container and the preservative solution according to the present invention, because the above-mentioned configuration is provided, the storage stability of cfDNA contained in a cell-containing solution can be enhanced even when the cell-containing solution is stored under a room temperature environment. In the storage container and the preservative solution according to the present invention, the storage stability of cfDNA contained in a cell-containing solution can be enhanced even when, for example, the cell-containing solution is stored under an environment having a temperature of 15°C or higher and 25°C or lower. In the present invention, it becomes possible to effectively prevent cells contained in the cell-containing solution from disruption or death during the storage of the cell-containing solution under a room temperature environment, thereby preventing the contamination of the cell-containing solution by gDNA in the cells. Accordingly, in the present invention, the storage stability of cfDNA can be enhanced. In the present invention, it becomes possible to store cfDNA contained in a cell-containing solution, for example, at 25°C for 1 day or longer (e.g., 7 days) stably.

The cell-containing solution is a solution containing cells. An example of the cell-containing solution is a body fluid, more specifically blood, urine, cerebrospinal fluid or the like. In blood, leukocytes and others are contained as cells.

The cell-containing solution is preferably blood, because the effect of the present invention can be achieved more effectively.

The storage container according to the present invention may have the first configuration, or the second configuration, or both of the first configuration and the second configuration. The storage container according to the present invention may have only one of the first configuration and the second configuration, or both of the first configuration and the second configuration. From the viewpoint of achieving the effect of the present invention more effectively, it is preferred that the storage container according to the present invention has both of the first configuration and the second configuration.

The preservative solution according to the present invention may have the third configuration, or the fourth configuration, or both of the third configuration and the fourth configuration. The preservative solution according to the present invention may have only one of the third configuration and the fourth configuration, or both of the third configuration and the fourth configuration. From the viewpoint of achieving the effect of the present invention more effectively, it is preferred that the preservative solution according to the present invention have both of the third configuration and the fourth configuration.

### (Preservative solution)

The preservative solution has a liquid form at 25°C.

The preservative solution contains a cell-membrane-permeable compound that has a molecular weight of 100 or less and cannot be frozen at 0°C, or contains a cell-membrane-impermeable compound having a molecular weight of 300 or more. The preservative solution may contain the cell-membrane-permeable compound, or the cell-membrane-impermeable compound, or both of the cell-membrane-permeable compound and the cell-membrane-impermeable compound.

Whether a compound is a cell-membrane-permeable compound or a cell-membrane-impermeable compound can be generally determined depending on the molecular weight, electric charge, polarity or the like of the compound. A compound having no electric charge can permeate through a cell membrane due to the passive diffusion thereof, and is generally classified as a cell-membrane-permeable compound. A compound having an electric charge, for example, cannot permeate through a cell membrane due to the passive diffusion thereof, and is generally classified as a cell-membrane-impermeable compound.

### <Cell-membrane-permeable compound>

It is preferred that the preservative solution contains a cell-membrane-permeable compound that has a molecular weight of 100 or less and cannot be frozen at 0°C. The cell-membrane-permeable compound is a compound which can permeate through a cell membrane. When the storage container has the first configuration, the preservative solution contains the cell-membrane-permeable compound. When the preservative solution has the third configuration, the preservative solution contains the cell-membrane-permeable compound. When the cell-membrane-permeable compound is used, the inside of cells can be dehydrated satisfactorily, and the cell-membrane-permeable compound can be retained in the cells satisfactorily. Therefore, it becomes possible to effectively prevent the cell from the disruption or death during storage, thereby effectively preventing the contamination of the cell-containing solution by gDNA occurring in the cells. The cell-membrane-permeable compound is also known as a cryoprotective agent for cells. Only one type of the cell-membrane-permeable compound may be used, or two or more types of the cell-membrane-permeable compounds may be used in combination.

Examples of the cell-membrane-permeable compound include ethylene glycol, propylene glycol, glycerin, dimethyl sulfoxide, acetamide, 1,3-propanediol and butylene glycol.

The cell-membrane-permeable compound is preferably ethylene glycol, propylene glycol, glycerin, dimethyl sulfoxide, acetamide, 1,3-propanediol or butylene glycol, more preferably ethylene glycol, propylene glycol, glycerin or dimethyl sulfoxide. The cell-membrane-permeable compound is preferably a polyhydric alcohol. In this case, the effect of the present invention can be achieved more effectively.

The content of the cell-membrane-permeable compound in the preservative solution is not particularly limited, as long as the content of the cell-membrane-permeable compound in the mixed solution X is 1 vol% or more and 5 vol% or less. The content of the cell-membrane-permeable compound in the preservative solution may be varied appropriately depending on the mixing ratio between the cell-containing solution and the preservative solution or the like.

The content of the cell-membrane-permeable compound in the preservative solution is preferably 2 vol% or more, more preferably 10 vol% or more, still more preferably 20 vol% or more, and preferably 50 vol% or less, more preferably 40 vol% or less, still more preferably 30 vol% or less. When the content of the cell-membrane-permeable compound is equal to or more than the lower limit and equal to or less than the upper limit, the content of the cell-containing solution in the mixed solution of the cell-containing solution and the preservative solution (i.e., a mixed solution X) can be adjusted to the above-mentioned range. As a result, the effect of the present invention can be achieved more effectively.

### <Cell-membrane-impermeable compound>

It is preferred that the preservative solution contains a cell-membrane-impermeable compound having a molecular weight of 300 or more. When the storage container has the second configuration, the preservative solution contains the cell-membrane-impermeable compound. When the preservative solution has the fourth configuration, the preservative solution contains the cell-membrane-impermeable compound. When the cell-membrane-impermeable compound is used, the aggregation of the cells can be prevented effectively, and the effect to protect cell membranes can be enhanced. Therefore, it becomes possible to effectively prevent the cell from the disruption or death during storage, thereby effectively preventing the contamination of the cell-containing solution by gDNA occurring in the cells. Only one type of the cell-membrane-impermeable compound may be used, or two or more types of the cell-membrane-impermeable compounds may be used in combination.

Examples of the cell-membrane-impermeable compound include polyvinylpyrrolidone, polyethylene glycol, polyvinyl alcohol, a polysaccharide, a derivative of a polysaccharide, a sugar alcohol and Ficoll. Examples of the polysaccharide include cellulose, sucrose, and dextran. An example of the derivative of a polysaccharide is hydroxypropyl cellulose.

From the viewpoint of achieving the effect of the present invention more effectively, it is preferred that the cell-membrane-impermeable compound is polyvinylpyrrolidone, polyethylene glycol, polyvinyl alcohol, a polysaccharide, a derivative of a polysaccharide, a sugar alcohol or Ficoll. From the viewpoint of achieving the effect of the present invention more effectively, the cell-membrane-impermeable compound is more preferably polyvinylpyrrolidone, polyethylene glycol, polyvinyl alcohol, dextran or hydroxypropyl cellulose, and further preferably polyvinylpyrrolidone, polyethylene glycol or polyvinyl alcohol.

From the viewpoint of achieving the effect of the present invention, the molecular weight of the cell-membrane-impermeable compound is 300 or more. When the structural formula of the cell-membrane-impermeable compound can be identified, the molecular weight of the cell-membrane-impermeable compound refers to a molecular weight calculated from the structural formula. When the structural formula cannot be identified, the molecular weight refers to a weight average molecular weight.

The molecular weight (weight average molecular weight) of the cell-membrane-impermeable compound is preferably 1000 or more, more preferably 10,000 or more, still more preferably 100,000 or more, and preferably 4,000,000 or less, more preferably 1,000,000 or less. When the molecular weight (weight average molecular weight) of the cell-membrane-impermeable compound is equal to or more than the lower limit and equal to or less than the upper limit, the effect of the present invention can be achieved more effectively.

The weight average molecular weight refers to a weight average molecular weight which is determined in terms of polystyrene by gel permeation chromatography (GPC).

The content of the cell-membrane-impermeable compound in the preservative solution is not particularly limited, as long as the content of the cell-membrane-impermeable compound in the mixed solution X becomes 0.5 µmol/L or more and 5 µmol/L or less. The content of the cell-membrane-impermeable compound in the preservative solution may be varied appropriately depending on the mixing ratio between the cell-containing solution and the preservative solution or the like.

The content of the cell-membrane-impermeable compound in the preservative solution is preferably 1.0 µmol/L or more, more preferably 5 µmol/L or more, and preferably 100 µmol/L or less, more preferably 50 µmol/L or less. When the content of the cell-membrane-impermeable compound is equal to or more than the lower limit and equal to or less than the upper limit, the content of the cell-membrane-impermeable compound in the mixed solution of the cell-containing solution and the preservative solution (i.e., a mixed solution X) can be adjusted to the above-mentioned range easily. As a result, the effect of the present invention can be achieved more effectively.

### <Formaldehyde donor compound>

It is preferred that the preservative solution contains a formaldehyde donor compound as a compound that is different from either of the cell-membrane-permeable compound and the cell-membrane-impermeable compound. The formaldehyde donor compound is a compound that can release formaldehyde therefrom. When the formaldehyde donor compound is used, a membrane protein in cells can be crosslinked by the action of formaldehyde released from the formaldehyde donor compound upon the mixing of the cell-containing solution with the preservative solution, thereby enhancing the stability of the cells. In conventional preservative solutions each containing a formaldehyde donor compound, the fragment length of cfDNA contained in the cell-containing solution is likely to be changed during the storage of the cell-containing solution under a room temperature environment. In contrast, in the present invention, it becomes possible to make the change in fragment length of cfDNA unlikely to occur even when the preservative solution contains a formaldehyde donor compound. Only one type of the formaldehyde donor compound may be used, or two or more types of the formaldehyde donor compounds may be used in combination.

Examples of the formaldehyde donor compound include a hydantoin compound, imidazolidinylurea, diazolidinylurea, hexamethylenetetramine, N,N"-methylenebis-[N'-(3-hydroxymethyl-2,5-diaoxo-4-imidazolidinyl)urea], a tertiary amine compound, a secondary amine compound and a primary amine compound.

Examples of the hydantoin compound include DMDM hydantoin, 1-hydroxymethyl-5,5-dimethylhydantoin, 1,3-dimethylol-5,5-hydantoin and 1,3-dichloro-5,5-dimethylhydantoin.

From the viewpoint of achieving the effect of the present invention more effectively, the formaldehyde donor compound is preferably a hydantoin compound, more preferably DMDM hydantoin or 1-hydroxymethyl-5,5-dimethylhydantoin.

The content of the formaldehyde donor compound in the preservative solution is not particularly limited. The content of the formaldehyde donor compound in the preservative solution may be varied appropriately in such a manner that, for example, the content of the formaldehyde donor compound in the below-mentioned mixed solution Y can satisfy the below-mentioned range. The content of the formaldehyde donor compound in the preservative solution may be varied appropriately depending on the mixing ratio between the cell-containing solution and the preservative solution, the type of the formaldehyde donor compound, or the like.

### <Osmotic pressure-controlling agent>

It is preferred that the preservative solution contains an osmotic pressure-controlling agent as a compound that is different from either of the cell-membrane-permeable compound and the cell-membrane-impermeable compound. When the osmotic pressure-controlling agent is used, it becomes possible to increase the osmotic pressure in the mixed solution effectively. Only one type of the osmotic pressure-controlling agent may be used, or two or more types of the osmotic pressure-controlling agents may be used in combination.

Examples of the osmotic pressure-controlling agent include: an inorganic ion such as sodium chloride and potassium chloride; and a sugar such as glucose and sucrose.

From the viewpoint of achieving the effect of the present invention more effectively, the osmotic pressure-controlling agent is preferably glucose, sucrose or sodium chloride, more preferably glucose or sodium chloride.

The content of the osmotic pressure-controlling agent in the preservative solution is not particularly limited. The content of the osmotic pressure-controlling agent in the preservative solution may be varied appropriately in such a manner that, for example, the osmotic pressure in the below-mentioned mixed solution Z satisfies the below-mentioned range.

### <Buffering agent>

It is preferred that the preservative solution contains a buffering agent as a compound that is different from either of the cell-membrane-permeable compound and the cell-membrane-impermeable compound. Only one type of the buffering agent may be used, or two or more types of the buffering agents may be used in combination.

Examples of the buffering agent include: glycine; sodium citrate; a phosphoric acid salt such as sodium phosphate, disodium hydrogen phosphate, sodium dihydrogen phosphate, potassium phosphate, dipotassium hydrogen phosphate and potassium dihydrogen phosphate; a carbonic acid salt such as sodium carbonate and sodium hydrogen carbonate; a boric acid salt such as sodium borate; carboxylic acid; dicarboxylic acid; a carboxylic acid derivative; a hydroxy carboxylic acid; aniline; an aniline derivative; an amino acid; an amine compound; an imidazole compound; an alcohol compound; ethylenediaminetetraacetic acid; pyrophosphoric acid; pyridine; cacodylic acid; glycerol phosphate; 2,4,6-collidine; N-ethylmorpholinel, morpholine; 4-aminopyridine; ammonia; ephedrine; hydroxyproline; piperidine and tris(hydroxymethyl)aminomethane.

From the viewpoint of achieving the buffering effect effectively, it is preferred that the buffering agent contains glycine, and it is preferred that the buffering agent is glycine.

The content of the buffering agent in the preservative solution is not particularly limited, as long as the buffering effect can be achieved. The content of the buffering agent in the preservative solution may be varied appropriately depending on the mixing ratio between the cell-containing solution and the preservative solution or the like.

### <Compound having chelating activity>

It is preferred that the preservative solution contains a compound having a chelating activity as a compound that is different from either of the cell-membrane-permeable compound and the cell-membrane-impermeable compound. A DNase is activated with a magnesium ion. Therefore, when a compound having a chelating activity is used, it becomes possible to prevent the decomposition of cfDNA in the cell-containing solution effectively. When the cell-containing solution is blood, it is preferred that the preservative solution contains an anticoagulant agent as the compound having a chelating activity. In this case, in addition to the effective prevention of the decomposition of cfDNA, it also becomes possible to effectively prevent the coagulation of blood during storage. Only one type of the compound having a chelating activity may be used, or two or more types of the compounds having a chelating activity may be used in combination.

Examples of the anticoagulant agent include ethylenediaminetetraacetic acid (EDTA), citric acid, and glycoletherdiamine tetraacetic acid (EGTA).

From the viewpoint of preventing the decomposition of cfDNA more effectively and when the cell-containing solution is blood, the anticoagulant agent preferably contains EDTA, and is preferably EDTA, from the viewpoint of preventing the coagulation of the blood during storage more effectively.

The content of the compound having a chelating activity in the preservative solution may be varied appropriately depending on the mixing ratio between the cell-containing solution and the preservative solution, the type of the compound having a chelating activity, or the like.

### <Other components>

The preservative solution may contain a component other than the cell-membrane-permeable compound, the cell-membrane-impermeable compound, the formaldehyde donor compound, the osmotic pressure-controlling agent, the buffering agent and the compound having a chelating activity. Examples of the above-mentioned other component include water and a pH adjusting agent.

It is preferred that the preservative solution contains water. The content of water in 100% by weight of the preservative solution is preferably 30% by weight or more, more preferably 40% by weight or more, and preferably 60% by weight or less, more preferably 50% by weight or less.

The osmotic pressure in the preservative solution is preferably 300 mOsm/L or more, more preferably 600 mOsm/L or more, and preferably 4000 mOsm/L or less, more preferably 3000 mOsm/L or less. When the osmotic pressure in the preservative solution is equal to or more than the lower limit and equal to or less than the upper limit, the effect of the present invention can be achieved more effectively.

The osmotic pressure in the preservative solution can be measured using an osmometer (e.g., "Osmometer 3250", manufactured by Advanced Instruments Inc.).

The pH value of the preservative solution is preferably 6.0 or more, more preferably 7.0 or more, and preferably 8.0 or less, more preferably 7.5 or less. When the pH value is equal to or more than the lower limit and equal to or less than the upper limit, the effect of the present invention can be achieved more effectively.

### (Storage container for cell-containing solutions)

The storage container according to the present invention is one which is intended to be used for the storage of a predetermined amount of a cell-containing solution. When the storage container according to the present invention has the first configuration, the storage container is configured such that, when a mixed solution X is prepared by collecting the predetermined amount of the cell-containing solution in the storage container and mixing the cell-containing solution with the preservative solution, the content of the cell-membrane-permeable compound in the mixed solution X falls within a specified range.

When the storage container according to the present invention has the second configuration, the storage container is configured such that, when a mixed solution X is prepared by collecting the predetermined amount of the cell-containing solution in the storage container and mixing the cell-containing solution with the preservative solution, the content of the cell-membrane-impermeable compound in the mixed solution X falls within a specified range.

In the storage container according to the present invention, the mixed solution X is prepared by mixing the predetermined amount of the cell-containing solution with the preservative solution contained in the storage container.

More specifically, the mixed solution X is prepared in the following manner.

A predetermined amount of a cell-containing solution to be collected in the storage container is collected in the storage container. For example, for a storage container in which 5 mL of blood is to be collected, 5 mL of blood is collected in the storage container. The predetermined amount of the cell-containing solution thus collected is mixed with the preservative solution by inversion mixing to produce a mixed solution X.

In the storage container having the first configuration, the content of the cell-membrane-permeable compound in the mixed solution X is 1 vol% or more and 5 vol% or less. That is, in the storage container having the first configuration, the content of the cell-membrane-permeable compound in 100% by volume of the mixed solution is 1 % by volume or more and 5% by volume or less. In the storage container having the first configuration, because the content of the cell-membrane-permeable compound in the mixed solution X falls within the above-mentioned range, water in cells can be dehydrated satisfactorily and the cell-membrane-permeable compound can be retained in the cells satisfactorily. Therefore, it becomes possible to effectively prevent the cell from the disruption or death during storage, thereby effectively preventing the contamination of the cell-containing solution by gDNA occurring in the cells.

In the mixed solution X, the content of the cell-membrane-permeable compound is preferably 2 vol% or more, and preferably 4 vol% or less. When the content of the cell-membrane-permeable compound is equal to or more than the lower limit and equal to or less than the upper limit, the inside of cells can be dehydrated more satisfactorily, and the cell-membrane-permeable compound can be retained in the cells more satisfactorily. Therefore, it becomes possible to more effectively prevent the cell from the disruption or death during storage, thereby more effectively preventing the contamination of the cell-containing solution by gDNA occurring in the cells.

In the storage container having the second configuration, the content of the cell-membrane-impermeable compound in the mixed solution X is 0.5 µmol/L or more and 5 µmol/L or less. In the storage container having the second configuration, because the content of the cell-membrane-impermeable compound in the mixed solution X falls within the above-mentioned range, the aggregation of cells can be prevented effectively, and the cell membrane protection effect can be enhanced. Therefore, it becomes possible to effectively prevent the cell from the disruption or death during storage, thereby effectively preventing the contamination of the cell-containing solution by gDNA occurring in the cells.

In the mixed solution X, the content of the cell-membrane-impermeable compound is preferably 1 µmol/L or more, and preferably 4 µmol/L or less. When the content of the cell-membrane-impermeable compound is equal to or more than the lower limit and equal to or less than the upper limit, the aggregation of cells can be prevented more effectively, and the cell membrane protection effect can be further enhanced. Therefore, it becomes possible to more effectively prevent the cell from the disruption or death during storage, thereby more effectively preventing the contamination of the cell-containing solution by gDNA occurring in the cells.

When the preservative solution contains a compound having a chelating activity such as EDTA, the content of the compound having a chelating activity or EDTA in the mixed solution X is preferably 4 mmol/L or more, more preferably 4.5 mmol/L or more, and preferably 7 mmol/L or less, more preferably 6.5 mmol/L or less. When the content of the compound having a chelating activity or the EDTA is equal to or more than the lower limit and equal to or less than the upper limit, the decomposition of cfDNA can be prevented more effectively. When the content of the EDTA is equal to or more than the lower limit and equal to or less than the upper limit, in the case where the cell-containing solution is blood, the coagulation of the blood during storage can be prevented more effectively.

When the preservative solution contains a buffering agent such as glycine, the content of the buffering agent or glycine in the mixed solution X is preferably 0.1 w/v% or more, more preferably 0.2 w/v% or more, and preferably 0.5 w/v% or less, more preferably 0.4 w/v% or less. When the content of the buffering agent or the glycine is equal to or more than the lower limit and equal to or less than the upper limit, the buffering effect can be achieved effectively.

In the storage container according to the present invention, when the preservative solution contains a formaldehyde donor compound, it is preferred that the content of the formaldehyde donor compound in the mixed solution Y satisfies a specified range.

Physiological saline (1 mL) is mixed with 7.4 g of sodium hypochlorite to prepare a solution Y', then the solution Y' in an amount that is the same as a predetermined amount of a cell-containing solution to be stored in the storage container is collected in the storage container, and then the solution Y' is mixed with the preservative solution to prepare a mixed solution Y. In the storage container according to the present invention, the mixed solution Y is prepared by mixing the predetermined amount of the solution Y' with the preservative solution contained in the storage container. The solution Y' is a solution that mimics a cell-containing solution.

More specifically, the mixed solution Y is prepared in the following manner.

The solution Y' in an amount that is the same as the predetermined amount of the cell-containing solution to be collected in the storage container is collected in the storage container. For example, for a storage container in which 5 mL of blood is to be collected, 5 mL of the solution Y' is collected in the storage container. The predetermined amount of the solution Y' thus collected is mixed with the preservative solution by inversion mixing to prepare a mixed solution Y.

When the preservative solution contains a formaldehyde donor compound, in the storage container according to the present invention, the content of formaldehyde in the mixed solution Y is preferably 100 mg/L or more, more preferably 110 mg/L or more. When the preservative solution contains a formaldehyde donor compound, in the storage container according to the present invention, the content of formaldehyde in the mixed solution Y is preferably 400 mg/L or less, more preferably 300 mg/L or less, still more preferably 200 mg/L or less, particularly preferably 190 mg/L or less. The mixed solution Y can contain formaldehyde as the result of the release of the formaldehyde from the formaldehyde donor compound. When the content of the formaldehyde in the mixed solution Y is equal to or more than the lower limit and equal to or less than the upper limit, it becomes possible to control the concentration of formaldehyde in the mixed solution of the cell-containing solution and the preservative solution adequately. Therefore, the stability of cells can be enhanced, and the decomposition of cfDNA can be prevented. If the content of formaldehyde in the mixed solution Y exceeds 400 mg/L, the crosslinking between cfDNA molecules or the crosslinking between a cfDNA molecule and a membrane protein is likely to occur compared to the case where the content of formaldehyde in the mixed solution Y is 400 mg/L or less and, as a result, the storage stability of cfDNA may be deteriorated.

The content of the formaldehyde in the mixed solution Y can be determined by a MBTH visual colorimetric method.

In the storage container according to the present invention, physiological saline in an amount that is the same as the predetermined amount of the cell-containing solution to be stored in the storage container is collected in the storage container, and the physiological saline is mixed with the preservative solution to prepare a mixed solution Z. The osmotic pressure in the mixed solution Z is preferably 300 mOsm/L or more, more preferably 350 mOsm/L or more, and preferably 1100 mOsm/L or less, more preferably 1000 mOsm/L or less. When the osmotic pressure in the mixed solution Z is equal to or more than the lower limit and equal to or less than the upper limit, it becomes possible to adjust the osmotic pressure in the mixed solution of the cell-containing solution and the preservative solution to a satisfactory level. As a result, the effect of the present invention can be achieved more effectively.

More specifically, the mixed solution Z is prepared in the following manner.

The physiological saline in an amount that is the same as the predetermined amount of the cell-containing solution to be collected in the storage container is collected in the storage container. For example, for a storage container in which 5 mL of blood is to be collected, 5 mL of physiological saline is collected in the storage container. The predetermined amount of physiological saline thus collected is mixed with the preservative solution by inversion mixing to prepare a mixed solution Z.

The osmotic pressure in the mixed solution Z can be measured using an osmometer (e.g., "Osmometer 3250", manufactured by Advanced Instruments Inc.).

### (Container main body)

The shape of the container main body is not particularly limited. The shape of the container main body is preferably a bottomed shape, more preferably a bottomed tubular shape.

The material for the container main body is not particularly limited. Examples of the material for the container main body include: a thermoplastic resin such as polyethylene, polypropylene, polystyrene, polyethylene terephthalate, polymethyl methacrylate and polyacrylonitrile; a heat-curable resin such as an unsaturated polyester resin, an epoxy resin and an epoxy-acrylate resin; a modified natural resin such as cellulose acetate, cellulose propionate, ethyl cellulose and ethylchitin; a silicate glass such as a soda-lime glass, a phosphosilicate glass and a borosilicate glass; and a glass such as a quartz glass. Only one type of the material for the container main body may be used, or two or more types of the materials for the container main body may be used in combination.

### (Plug body)

It is preferred that the storage container is equipped with a plug body. As the plug body, any conventional known plug body may be used. The plug body is preferably one which is made from a material and has a shape such that the plug body can be attached to an opening in the container main body air-tightly and liquid-tightly. When the cell-containing solution is blood, the plug body is preferably configured such that a blood collection needle can be pierced through the plug body.

Examples of the plug body include: a plug body that can fit in an opening in the container main body; and a sheet-like seal plug body.

The plug body may also be one which is equipped with a plug main body such as a rubber plug and a cap member made from a plastic material or the like. In this case, it becomes possible to eliminate the risk of the contact of blood with a human body upon the pulling of the plug body from the opening in the container main body after the collection of the blood.

Examples of the material for the plug body (or the plug main body) include a synthetic resin, an elastomer, a rubber and a metal foil. Examples of the rubber include a butyl rubber and a halogenated butyl rubber. An example of the metal foil is an aluminum foil. From the viewpoint of enhancing the air-tightness, the material for the plug body is preferably a butyl rubber. The plug body is preferably a butyl rubber plug.

### (Other detains about storage container and preservative solution)

The storage container is a container which is intended to store a predetermined amount of a cell-containing solution therein. The storage container is a container which is intended to collect and store a predetermined amount of a cell-containing solution therein. The storage container is a container which is intended to store the cell-containing solution and the preservative solution in a mixed state. The predetermined amount of the cell-containing solution can be varied appropriately depending on the size of the storage container or the like. The predetermined amount of the cell-containing solution is, for example, 1 mL, 5 mL, 10 mL or 20 mL.

The storage container is preferably a container which is intended to store the cell-containing solution in a liquid state, and is preferably a container which is intended to store the cell-containing solution without freezing the cell-containing solution. The storage container is preferably a container which is intended to store the cell-containing solution at 0°C or higher, more preferably at 1°C or higher, still more preferably at 15°C or higher, particularly preferably at 18°C or higher. The storage container is preferably a container which is intended to store the cell-containing solution at 100°C or lower, more preferably at 50°C or lower, still more preferably at 25°C or lower, particularly preferably at 24°C or lower.

The preservative solution is preferably one which is intended to store the cell-containing solution at 0°C or higher, more preferably at 1°C or higher, still more preferably at 15°C or higher, particularly preferably at 18°C or higher. The preservative solution is preferably one which is intended to store the cell-containing solution at 100°C or lower, more preferably at 50°C or lower, still more preferably at 25°C or lower, particularly preferably at 24°C or lower.

The content of the preservative solution to be contained in the container main body per 1 mL of the cell-containing solution to be stored is preferably 0.05 mL or more, more preferably 0.1 mL or more, and preferably 1 mL or less, more preferably 0.5 mL or less. When the content of the preservative solution is equal to or more than the lower limit and equal to or less than the upper limit, the effect of the present invention can be achieved more effectively without causing the excessive dilution of the cell-containing solution.

The preservative solution is preferably mixed with the cell-containing solution to be stored in an amount of 0.05 mL or more, more preferably 0.1 mL or more, and preferably 1 mL or less, more preferably 0.5 mL or less, per 1 mL of the cell-containing solution. In this case, the effect of the present invention can be achieved more effectively without causing the excessive dilution of the cell-containing solution.

The inner pressure in the storage container is not particularly limited. The storage container may be used as a vacuum blood collection tube in which the inside thereof is deaerated and which is hermetically sealed with the sealing member. When the storage container is a vacuum blood collection tube, it becomes possible to perform the collection of a certain amount of blood easily regardless of the levels of the skills of blood collecting persons.

From the viewpoint of preventing bacterial infections, it is preferred that the inside of the storage container is sterilized in accordance with the standards of ISO and JIS.

Hereinbelow, the present invention is described in more detail with reference to examples. However, the present invention is not limited to the following examples.

As the materials for the preservative solution, the following materials were provided.

### (Cell-membrane-permeable compounds)

Propylene glycol
Glycerin
Dimethyl sulfoxide

### (Cell-membrane-impermeable compounds)

Polyethylene glycol (weight average molecular weight: 500,000)
Polyvinylpyrrolidone (weight average molecular weight: 30,000)
Dextran (weight average molecular weight: 200,000)

### (Formaldehyde donor compound)

### 1-Hydroxymethyl-5,5-dimethylhydantoin

(Osmotic pressure-controlling agent)
Glucose

### (Anticoagulant agent)

### EDTA•2Na

### (Buffering agent)

### Glycine

### (Other ingredient)

### Water

### (Examples 1 to 15)

### Preparation of preservative solution:

The components shown in Tables 1 to 4 were blended in the blending amounts shown in Tables 1 to 4 to prepare preservative solutions.

### Production of storage container:

As the container main body, a bottomed polyethylene terephthalate tube (a bottomed PET tube) was provided, which had a length of 100 mm and had an opening with an inner diameter of 14 mm. The prepared preservative solution (0.5 mL) was placed in the bottomed PET tube. The pressure in the inside of the storage container was reduced to 50 kPa, and was hermetically sealed with a butyl plug. In this manner, a storage container for storing 5 mL of blood therein was produced.

### (Comparative Examples 1 and 4)

A vacuum blood collection tube ("INSEPACK II-D" manufactured by Sekisui Medical Co., Ltd.) having EDTA contained therein was used as a storage container. In the vacuum blood collection tube, a cell-membrane-permeable compound, a cell-membrane-impermeable compound and a formaldehyde donor compound were not contained.

### (Comparative Examples 2 and 3)

Preservative solutions and storage containers were produced in the same manner as in Example 1, except that the types and contents of the components were changed to those shown in Table 1.

### (Evaluation)

### (1) Contents of cell-membrane-permeable compound, cell-membrane-impermeable compound, formaldehyde donor compound, osmotic pressure-controlling agent, anticoagulant agent and buffering agent in mixed solution X

Blood (5 mL) was added to each of the storage containers produced in Examples 1 to 15 and Comparative Examples 2 and 3, and the blood was mixed with the preservative solution by inversion mixing to prepare a mixed solution X. The contents of a cell-membrane-permeable compound, a cell-membrane-impermeable compound, a formaldehyde donor compound, an osmotic pressure-controlling agent, an anticoagulant agent and a buffering agent in the mixed solution X were determined.

### (2) Content of formaldehyde in mixed solution Y

A solution Y' was prepared by mixing 7.4 g of sodium hypochlorite with 1 L of physiological saline. The solution Y' (5 mL) was added to each of the storage containers produced in Examples 1 and 2 and Comparative Examples 2 and 3, and the solution Y' was mixed with the preservative solution by inversion mixing to prepare a mixed solution Y. The content of formaldehyde in the mixed solution Y was determined by a MBTH visual colorimetric method.

### (3) Osmotic pressure in mixed solution Z

Physiological saline (5 mL) was added to each of the storage containers produced in Examples 1 to 15 and Comparative Example 2, and the physiological saline was mixed with the preservative solution by inversion mixing to prepare a mixed solution Z. The osmotic pressure in the mixed solution Z was measured using an osmometer (e.g., "Osmometer 3250", manufactured by Advanced Instruments Inc.).

### (4) Storage stability of cfDNA

### (4-1) Storage stability of cfDNA on day 7 of storage at room temperature (Examples 1 and 2 and Comparative Examples 1 to 3)

Blood (5 mL) was collected in each of the storage containers produced in Examples 1 and 2 and Comparative Examples 1 to 3, and the blood was mixed with the preservative solution by inversion mixing. Subsequently, the storage container was allowed to stand under an environment having a temperature of 15°C to 25°C. A sample of the mixed solution of the blood and the preservative solution was collected from the storage container immediately after the storage, on day 3 of the storage and on day 7 of the storage. Plasma was collected from the collected mixed solution sample by centrifugation. DNA contained in the collected plasma was purified using a cfDNA purification kit ("QIAamp Circulating Nucleic Acid Kit" manufactured by QIAGEN).

The purified DNA was fluorescently labeled and was then subjected to electrophoresis using an electrophoresis system ("Agilent 2100 Bioanalyzer", and "High Sensivity DNA kit" manufactured by Agilent). An electrophoretic image was subjected to an image analysis, and the peak shape of cfDNA having a length of around 180 bp and the peak shape of gDNA having a length of 300 bp or more were observed.

### (4-2) Storage stability of cfDNA on day 1 of storage at room temperature (Examples 3 and 12 and Comparative Example 4)

The test was performed in the same manner as that employed in "(4-1) Storage stability of cfDNA on day 7 of storage at room temperature", except that the storage containers produced in Examples 3 to 12 and Comparative Example 4 were used, each of the storage containers was allowed to stand under an environment having a temperature of 15°C to 25°C for 1 day, and a sample of a mixed solution of blood and the preservative solution was collected.

### (4-3) Storage stability of cfDNA on day 4 of storage at room temperature (Examples 13 to 15)

The test was performed in the same manner as that employed in "(4-1) Storage stability of cfDNA on day 7 of storage at room temperature", except that the storage containers produced in Examples 13 to 15 were used, each of the storage containers was allowed to stand under an environment having a temperature of 15°C to 25°C for 3 days and for 4 days, and a sample of a mixed solution of blood and a preservative solution was collected.

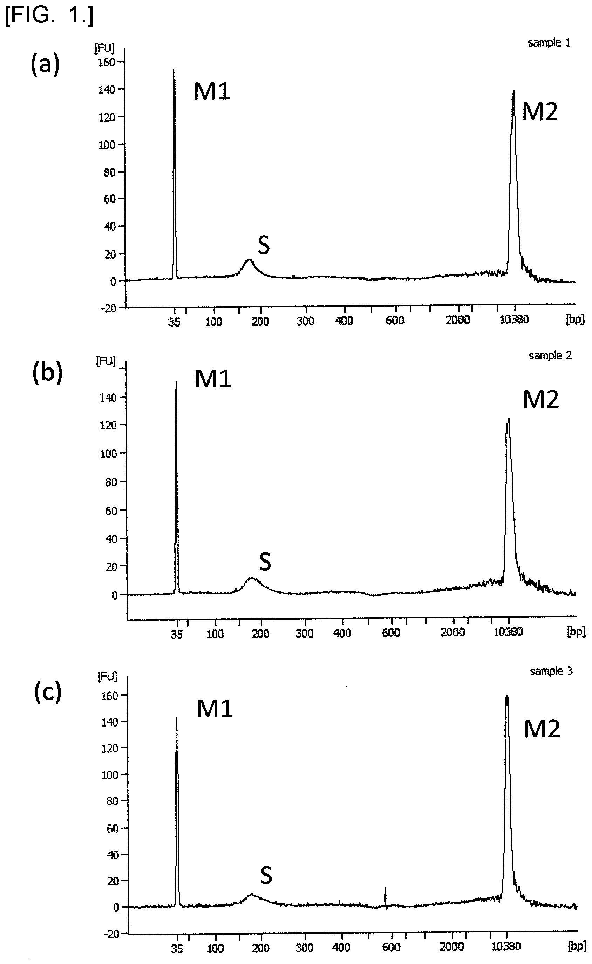

The compositions and the results are shown in Tables 1 to 4 and Figs. 1 to 11.

**[Table 1]**

| | | | Example 1 | Example 2 | Comparative Example 1 | Comparative Example 2 | Comparative Example 3 |
|---|---|---|---|---|---|---|---|
| Preservative solution | Cell-membrane-permeable compound | Propylene glycol | 33.0 vol% | 33.0 vol% | - | - | - |
| | | Glycerin | - | - | - | - | - |
| | | Dimethyl sulfoxide | - | - | - | - | - |
| | Cell-membrane-impermeable compound | Polyethylene glycol (MW: 500,000) | 11 µmol/L | 11 µmol/L | - | - | - |
| | | Polyvinylpyrrolidone (MW: 30,000) | - | - | - | - | - |
| | | Dextran (MW: 200,000) | - | - | - | - | - |
| | Formaldehyde donor compound | 1-Hydroxymethyl-5,5-dimethylhydantoin | 1.1 w/v% | 2.2 w/v% | - | 1.1 w/v% | 11.0 w/v% |
| | Osmotic pressure-controlling agent | Glucose | 20 w/v% | 20 w/v% | - | 20 w/v% | 20 w/v% |
| | Anticoagulant agent | EDTA | 55 mmol/L | 55 mmol/L | - | 55 mmol/L | 55 mmol/L |
| | Buffering agent | Glycine | 2.0 w/v% | 2.0 w/v% | - | 2.0 w/v% | 2.0 w/v% |
| | | Water | 67 v/v% | 67 v/v% | - | 100 v/v% | 67 v/v% |
| Mixed solution | Mixed solution X | Content of cell-membrane-permeable compound | 3.0 vol% | 3.0 vol% | - | - | - |
| | | Content of cell-membrane-impermeable compound | 1 µmol/L | 1 µmol/L | - | - | - |
| | | Content of formaldehyde donor compound | 0.1 w/v% | 0.2 w/v% | - | 0.1 w/v% | 1 w/v% |
| | | Content of glucose | 1.8 w/v% | 1.8 w/v% | - | 1.8 w/v% | 1.8 w/v% |
| | | Content of EDTA | 5.0 mmol/L | 5.0 mmol/L | 5.0 mmol/L | 5.0 mmol/L | 5.0 mmol/L |
| | | Content of glycine | 0.18 w/v% | 0.18 w/v% | - | 0.18 w/v% | 0.18 w/v% |
| | Mixed solution Y | Content of formaldehyde | 180 mg/mL | 274 mg/mL | - | 180 mg/mL | 683 mg/mL |
| | Mixed solution Z | Osmotic pressure | 896 mOsm/L | 898 mOsm/L | - | 499 mOsm/L | - |
| Storage stability of cfDNA | | | Fig. 1 | Fig. 2 | Figs. 3(a) to 3(c) | Fig. 4 | Fig. 5 |

**[Table 2]**

| | | | Example 3 | Example 4 | Example 5 | Example 6 | Example 7 | Example 8 |
|---|---|---|---|---|---|---|---|---|
| Preservative solution | Cell-membrane-permeable compound | Propylene glycol | 33.0 vol% | - | - | - | - | - |
| | | Glycerin | - | 33.0 vol% | - | - | - | - |
| | | Dimethyl sulfoxide | - | - | 33.0 vol% | - | - | - |
| | Cell-membrane-impermeable compound | Polyethylene glycol (MW: 500,000) | - | - | - | 11 µmol/L | - | - |
| | | Polyvinylpyrrolidone (MW: 30,000) | - | - | - | - | 11 µmol/L | - |
| | | Dextran (MW: 200,000) | - | - | - | - | - | 11 µmol/L |
| | Formaldehyde donor compound | 1-Hydroxymethyl-5,5-dimethylhydantoin | - | - | - | - | - | - |
| | Osmotic pressure-controlling agent | Glucose | 20 w/v% | 20 w/v% | 20 w/v% | 20 w/v% | 20 w/v% | 20 w/v% |
| | Anticoagulant agent | EDTA | 55 mmol/L | 55 mmol/L | 55 mmol/L | 55 mmol/L | 55 mmol/L | 55 mmol/L |
| | Buffering agent | Glycine | 2.0 w/v% | 2.0 w/v% | 2.0 w/v% | 2.0 w/v% | 2.0 w/v% | 2.0 w/v% |
| | | Water | 67 v/v% | 67 v/v% | 67 v/v% | 100 v/v% | 100 v/v% | 100 v/v% |
| Mixed solution | Mixed solution X | Content of cell-membrane-permeable compound | 3.0 vol% | 3.0 vol% | 3.0 vol% | - | - | - |
| | | Content of cell-membrane-impermeable compound | - | - | - | 1 µmol/L | 1 µmol/L | 1 µmol/L |
| | | Content of formaldehyde donor compound | - | - | - | - | - | - |
| | | Content of glucose | 1.8 w/v% | 1.8 w/v% | 1.8 w/v% | 1.8 w/v% | 1.8 w/v% | 1.8 w/v% |
| | | Content of EDTA | 5.0 mmol/L | 5.0 mmol/L | 5.0 mmol/L | 5.0 mmol/L | 5.0 mmol/L | 5.0 mmol/L |
| | | Content of glycine | 0.18 w/v% | 0.18 w/v% | 0.18 w/v% | 0.18 w/v% | 0.18 w/v% | 0.18 w/v% |
| | Mixed solution Y | Content of formaldehyde | - | - | - | - | - | - |
| | Mixed solution Z | Osmotic pressure | 908 mOsm/L | 877 mOsm/L | 895 mOsm/L | 398 mOsm/L | 409 mOsm/L | 404 mOsm/L |
| Storage stability of cfDNA | | | Fig. 6(a) | Fig. 6(b) | Fig. 6(c) | Fig. 7(a) | Fig. 7(b) | Fig. 7(c) |

**[Table 3]**

| | | | Example 9 | Example 10 | Example 11 | Example 12 | Comparative Example 4 |
|---|---|---|---|---|---|---|---|
| Preservative solution | Cell-membrane-permeable compound | Propylene glycol | 11.0 vol% | 55 vol% | - | - | - |
| | | Glycerin | - | - | - | - | - |
| | | Dimethyl sulfoxide | - | - | - | - | - |
| | Cell-membrane-impermeable compound | Polyethylene glycol (MW: 500,000) | - | - | 5.5 µmol/L | 55 µmol/L | - |
| | | Polyvinylpyrrolidone (MW: 30,000) | - | - | - | - | - |
| | | Dextran (MW: 200,000) | - | - | - | - | - |
| | Formaldehyde donor compound | 1-Hydroxymethyl-5,5-dimethylhydantoin | - | - | - | - | - |
| | Osmotic pressure-controlling agent | Glucose | 20 w/v% | 20 w/v% | 20 w/v% | 20 w/v% | - |
| | Anticoagulant agent | EDTA | 55 mmol/L | 55 mmol/L | 55 mmol/L | 55 mmol/L | - |
| | Buffering agent | Glycine | 2.0 w/v% | 2.0 w/v% | 2.0 w/v% | 2.0 w/v% | - |
| | | Water | 89 v/v% | 45 v/v% | 100 v/v% | 100 v/v% | - |
| Mixed solution | Mixed solution X | Content of cell-membrane-permeable compound | 1.0 vol% | 5.0 vol% | - | - | - |
| | | Content of cell-membrane-impermeable compound | - | - | 0.5 µmol/L | 5 µmol/L | - |
| | | Content of formaldehyde donor compound | - | - | - | - | - |
| | | Content of glucose | 1.8 w/v% | 1.8 w/% | 1.8 w/v% | 1.8 w/v% | - |
| | | Content of EDTA | 5.0 mmol/L | 5.0 mmol/L | 5.0 mmol/L | 5.0 mmol/L | 5.0 mmol/L |
| | | Content of glycine | 0.18 w/v% | 0.18 w/v% | 0.18 w/v% | 0.18 w/v% | - |
| | Mixed solution Y | Content of formaldehyde | - | - | - | - | - |
| | Mixed solution Z | Osmotic pressure | 557 mOsm/L | 1060 mOsm/L | 397 mOsm/L | 380 mOsm/L | - |
| Storage stability of cfDNA | | | Fig. 8(a) | Fig. 8(b) | Fig. 8(c) | Fig. 8(d) | Fig. 3(d) |

**[Table 4]**

| | | | Example 13 | Example 14 | Example 15 |
|---|---|---|---|---|---|
| Preservative solution | Cell-membrane-permeable compound | Propylene glycol | 33.0 vol% | - | - |
| | | Glycerin | - | 33.0 vol% | - |
| | | Dimethyl sulfoxide | - | - | 33.0 vol% |
| | Cell-membrane-impermeable compound | Polyethylene glycol (MW: 500,000) | 11 µmol/L | - | - |
| | | Polyvinylpyrrolidone (MW: 30,000) | - | 11 µmol/L | - |
| | | Dextran (MW: 200,000) | - | - | 11 µmol/L |
| | Formaldehyde donor compound | 1-Hydroxymethyl-5,5-dimethylhydantoin | - | - | - |
| | Osmotic pressure-controlling agent | Glucose | 20 w/v% | 20 w/v% | 20 w/v% |
| | Anticoagulant agent | EDTA | 55 mmol/L | 55 mmol/L | 55 mmol/L |
| | Buffering agent | Glycine | 2.0 w/v% | 2.0 w/v% | 2.0 w/v% |
| | | Water | 67 v/v% | 67 v/v% | 67 v/v% |
| Mixed solution | Mixed solution X | Content of cell-membrane-permeable compound | 3.0 vol% | 3.0 vol% | 3.0 vol% |
| | | Content of cell-membrane-impermeable compound | 1 µmol/L | 1 µmol/L | 1 µmol/L |
| | | Content of formaldehyde donor compound | - | - | - |
| | | Content of glucose | 1.8 w/v% | 1.8 w/v% | 1.8 w/v% |
| | | Content of EDTA | 5.0 mmol/L | 5.0 mmol/L | 5.0 mmol/L |
| | | Content of glycine | 0.18 w/v% | 0.18 w/v% | 0.18 w/v% |
| | Mixed solution Y | Content of formaldehyde | - | - | - |
| | Mixed solution Z | Osmotic pressure | 1025 mOsm/L | 886 mOsm/L | 1036 mOsm/L |
| Storage stability of cfDNA | | | Fig. 9 | Fig. 10 | Fig. 11 |

In Figs. 1 to 11, the transverse axis refers to the number of base pairs (bp) of DNA, and the vertical axis refers to a fluorescence intensity. In Figs. 1 to 11, peaks indicated by M1 and M2 represent peaks for markers, and a peak indicated by S represents a peak coming from cfDNA.

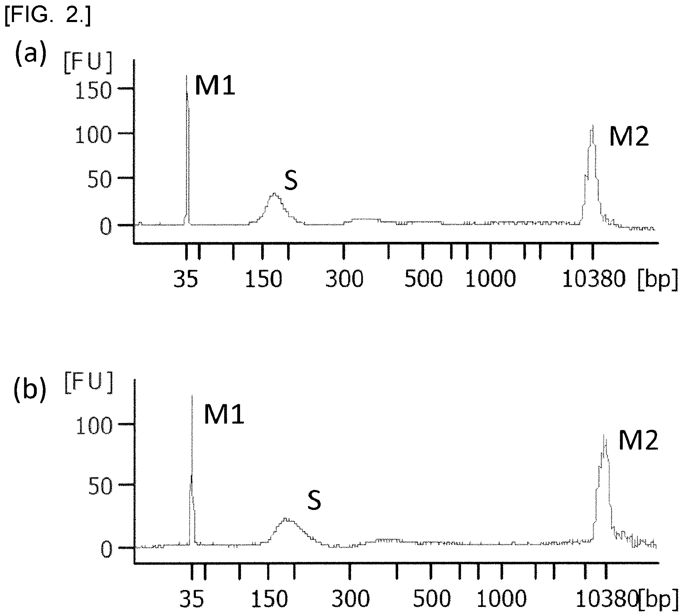

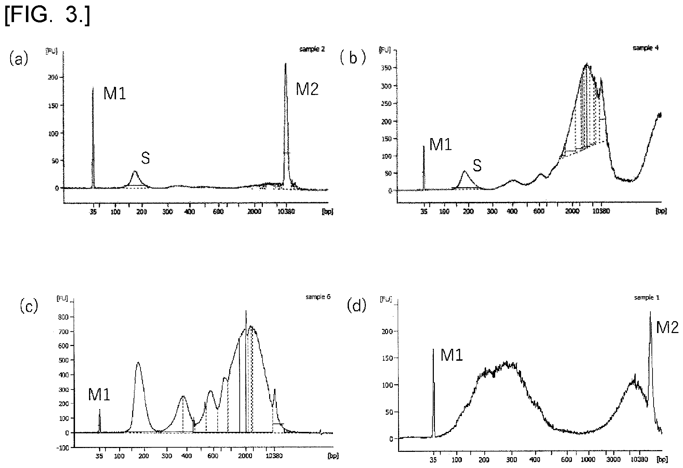

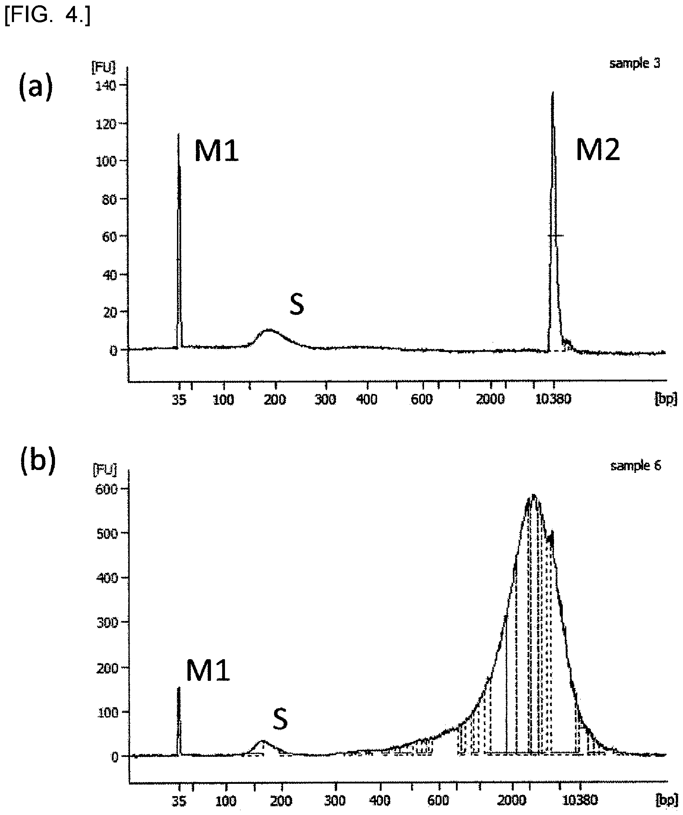

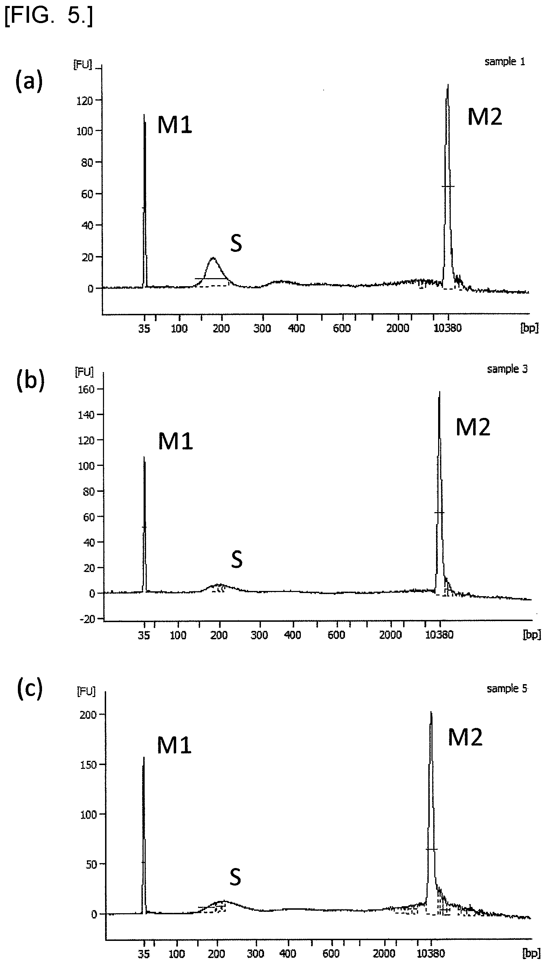

Figs. 1(a) to 1(c) show the results of the image analysis of electrophoretic images obtained in the evaluation of storage stability of cfDNA in Example 1. Figs. 2(a) and 2(b) show the results of the image analysis of electrophoretic images obtained in the evaluation of storage stability of cfDNA in Example 2. Figs. 3(a) to 3(c) show the results of the image analysis of electrophoretic images obtained in the evaluation of storage stability of cfDNA in Comparative Example 1. Figs. 4(a) and 4(b) show the results of the image analysis of electrophoretic images obtained in the evaluation of storage stability of cfDNA in Comparative Example 2. Figs. 5(a) to 5(c) show the results of the image analysis of electrophoretic images obtained in the evaluation of storage stability of cfDNA in Comparative Example 3. Figs. 1(a), 2(a), 3(a), 4(a) and 5(a) show the results each of which was obtained using a mixed solution immediately after the collection of blood in the storage container. Figs. 1(b), 3(b), 4(b) and 5(b) show the results each of which was obtained using a mixed solution on day 3 of the storage of blood in the storage container. Figs. 1(c), 2(b), 3(c) and 5(c) show the results each of which was obtained using a mixed solution on day 7 of the storage of blood in the storage container.

As shown in Figs. 1 and 2, with respect to purified DNA molecules respectively obtained in Examples 1 and 2, any peak other than a peak coming from cfDNA which appears around 180 bp was not confirmed until day 7 of the storage. The shape of the peak coming from cfDNA was well retained over 7 days of the storage under an environment having a temperature of 15°C to 25°C. Therefore, it is understood that the storage stability of cfDNA was enhanced.

On the other hand, as shown in Figs. 3 and 4, with respect to purified DNA molecules obtained in Comparative Examples 1 and 2, peaks coming from a molecule having a length of 300 bp or more which was different from cfDNA was detected. It is considered that the peaks were associated with the contamination of blood with gDNA from leukocytes or the like.

As shown in Fig. 5, with respect to purified DNA obtained in Comparative Example 3, although the contamination with gDNA from leukocytes or the like was reduced to a certain extent, the peak for cfDNA became broader. Therefore, it is understood that the fluorescence intensity was decreased. This phenomenon is assumed to occur as the result of the crosslinking between cfDNA molecules which was induced by formaldehyde.

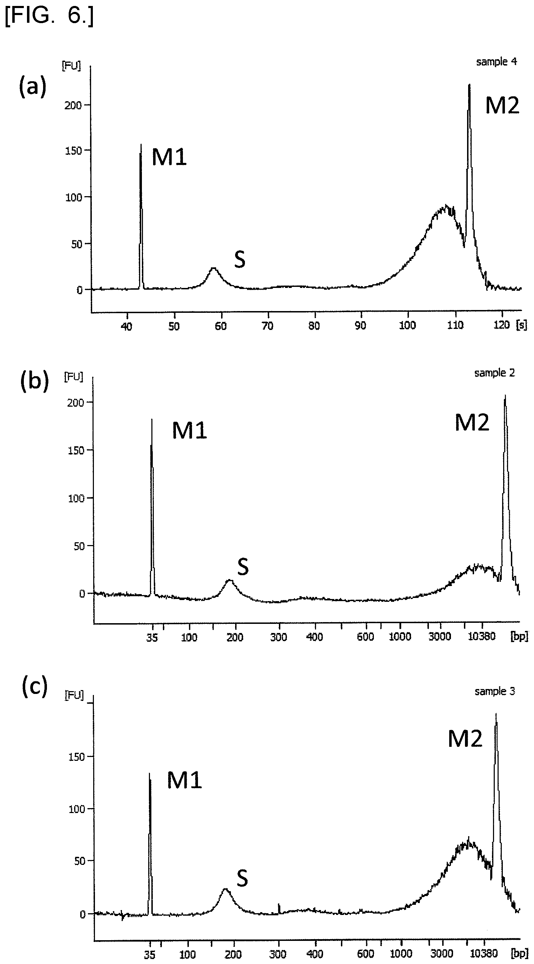

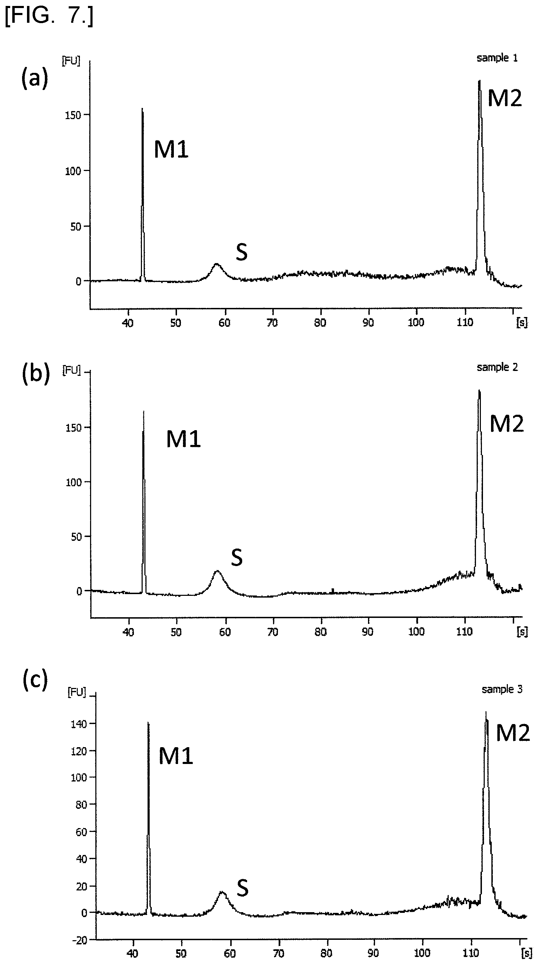

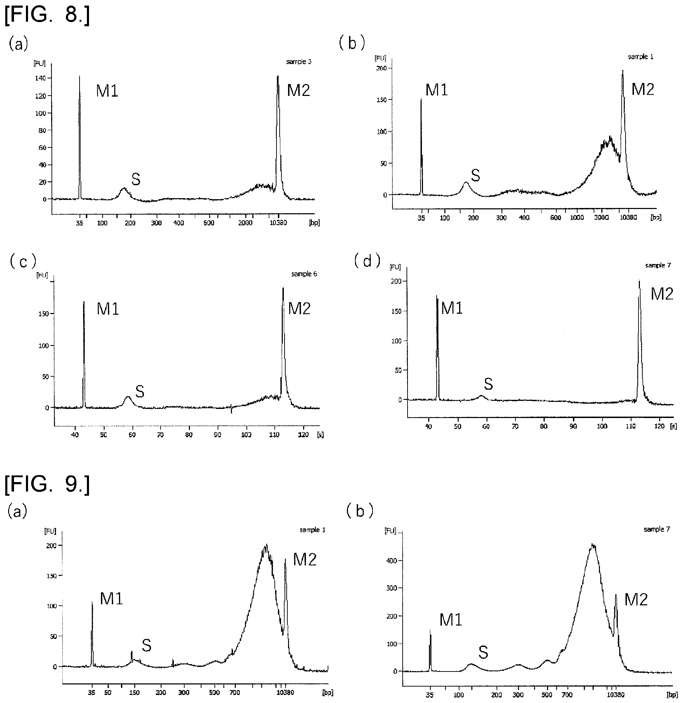

Figs. 6(a) to 6(c) show the results of the image analysis of electrophoretic images obtained in the evaluation of the storage stability of cfDNA in Examples 3 to 5, respectively. Figs. 7(a) to 7(c) show the results of the image analysis of electrophoretic images obtained in the evaluation of the storage stability of cfDNA in Examples 6 to 8, respectively. Figs. 8(a) to 8(d) show the results of the image analysis of electrophoretic images obtained in the evaluation of the storage stability of cfDNA in Examples 9 to 12, respectively. Fig. 3(d) shows the result of the image analysis of an electrophoretic image obtained in the evaluation of storage stability of cfDNA in Comparative Example 4. Figs. 6(a) to 6(c), 7(a) to 7(c), 8(a) to 8(d) and 3(d) show the results each of which was obtained using a mixed solution 1 day after blood was stored in the storage container.

As shown in Fig. 6, in Examples 3 to 5, although a peak associated with the contamination of blood with gDNA from leukocytes or the like was detected, the storage stability of cfDNA was enhanced compared with Comparative Example 4.

As shown in Fig. 7, in Examples 6 to 8, the storage stability of cfDNA was enhanced.

As shown in Figs. 8(a) and 8(b), in Examples 9 and 10, the storage stability of cfDNA was enhanced compared with Comparative Example 4.

As shown in Figs. 8(c) and 8(d), in Examples 11 and 12, the storage stability of cfDNA was enhanced.

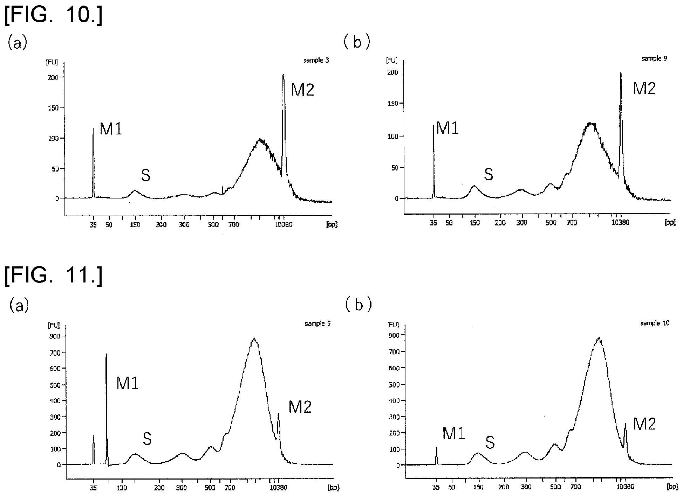

Figs. 9(a) and 9(b) show the results of the image analysis of electrophoretic images obtained in the evaluation of storage stability of cfDNA in Example 13. Figs. 10(a) and 10(b) show the results of the image analysis of electrophoretic images obtained in the evaluation of storage stability of cfDNA in Example 14. Figs. 11(a) and 11(b) show the results of the image analysis of electrophoretic images obtained in the evaluation of storage stability of cfDNA in Example 15. Figs. 9(a), 10(a) and 11(a) show the results each of which was obtained using a mixed solution on day 3 of the storage of blood in the storage container. Figs. 9(b), 10(b) and 11(b) show the results each of which was obtained using a mixed solution on day 4 of the storage of blood in the storage container.

### EXPLANATION OF SYMBOLS

M1, M2: Maker
S: cfDNA

## Claims

1. A storage container for cell-containing solutions, which is used for storing a predetermined amount of a cell-containing solution,
the storage container being provided with a container main body and a preservative solution contained in the container main body,
wherein the storage container has a first configuration such that the preservative solution contains a cell-membrane-permeable compound that has a molecular weight of 100 or less and cannot be frozen at 0°C and, when a mixed solution X is prepared by collecting the predetermined amount of the cell-containing solution in the storage container for cell-containing solutions and mixing the cell-containing solution with the preservative solution, the content of the cell-membrane-permeable compound in the mixed solution X is 1 vol% or more and 5 vol% or less, or
the storage container has a second configuration such that the preservative solution contains a cell-membrane-impermeable compound having a molecular weight of 300 or more and, when a mixed solution X is prepared by collecting the predetermined amount of the cell-containing solution in the storage container for cell-containing solutions and mixing the cell-containing solution with the preservative solution, the content of the cell-membrane-impermeable compound in the mixed solution X is 0.5 µmol/L or more and 5 µmol/L or less.

2. The storage container for cell-containing solutions according to claim 1, wherein the storage container has the first configuration, and the cell-membrane-permeable compound is a polyhydric alcohol.

3. The storage container for cell-containing solutions according to claim 1, wherein the storage container has the first configuration, and the cell-membrane-permeable compound is ethylene glycol, propylene glycol, glycerin, dimethyl sulfoxide, acetamide, 1,3-propanediol or butylene glycol.

4. The storage container for cell-containing solutions according to any one of claims 1 to 3, wherein the storage container has the second configuration, and the cell-membrane-impermeable compound is a compound having a molecular weight of 1000 or more.

5. The storage container for cell-containing solutions according to any one of claims 1 to 3, wherein the storage container has the second configuration, and the cell-membrane-impermeable compound is polyvinylpyrrolidone, polyethylene glycol, polyvinyl alcohol, a polysaccharide, a derivative of a polysaccharide, a sugar alcohol or Ficoll.

6. The storage container for cell-containing solutions according to any one of claims 1 to 5, wherein the storage container has the first configuration, and the preservative solution contains a formaldehyde donor compound as a compound that is different from either of the cell-membrane-permeable compound and the cell-membrane-impermeable compound.

7. The storage container for cell-containing solutions according to any one of claims 1 to 6, wherein
the preservative solution contains a formaldehyde donor compound as a compound that is different from either of the cell-membrane-permeable compound and the cell-membrane-impermeable compound, and
when a solution Y' is prepared by mixing 7.4 g of sodium hypochlorite with 1 L of physiological saline and a mixed solution Y is prepared by collecting the solution Y' in an amount equal to the predetermined amount of the cell-containing solution to be stored in the storage container for cell-containing solutions in the storage container for cell-containing solutions and mixing the solution Y' with the preservative solution, the content of formaldehyde in the mixed solution Y is 100 mg/L or more and 400 mg/L or less.

8. The storage container for cell-containing solutions according to claim 6 or 7, wherein the formaldehyde donor compound is DMDM hydantoin or 1-hydroxymethyl-5,5-dimethylhydantoin.

9. The storage container for cell-containing solutions according to any one of claims 1 to 8, wherein, when a mixed solution Z is prepared by collecting physiological saline in an amount equal to the predetermined amount of the cell-containing solution to be stored in the storage container for cell-containing solutions in the storage container for cell-containing solutions and mixing the physiological saline with the preservative solution, the osmotic pressure in the mixed solution Z is 300 mOsm/L or more and 1100 mOsm/L or less.

10. The storage container for cell-containing solutions according to any one of claims 1 to 9, wherein
the preservative solution contains an osmotic pressure-controlling agent as a compound that is different from either of the cell-membrane-permeable compound and the cell-membrane-impermeable compound, and
the osmotic pressure-controlling agent is glucose or sodium chloride.

11. The storage container for cell-containing solutions according to any one of claims 1 to 10, wherein
the preservative solution contains a compound having a chelating activity as a compound that is different from either of the cell-membrane-permeable compound and the cell-membrane-impermeable compound,
the compound having a chelating activity comprises EDTA; and
the content of the EDTA in the mixed solution X is 4 mmol/L or more and 7 mmol/L or less.

12. The storage container for cell-containing solutions according to any one of claims 1 to 11, wherein
the preservative solution contains a buffering agent as a compound that is different from either of the cell-membrane-permeable compound and the cell-membrane-impermeable compound,
the buffering agent comprises glycine, and
the content of the glycine in the mixed solution X is 0.5 w/v% or less.

13. The storage container for cell-containing solutions according to any one of claims 1 to 12, wherein the storage container has the first configuration and the second configuration.

14. The storage container for cell-containing solutions according to any one of claims 1 to 13, wherein the cell-containing solution is blood.

15. A preservative solution for use in the storage of a cell-containing solution,
the preservative solution having a third configuration such that a cell-membrane-permeable compound that has a molecular weight of 100 or less and cannot be frozen at 0°C is contained, in which the content of the cell-membrane-permeable compound is 2 vol% or more and 50 vol% or less, or
the preservative solution having a fourth configuration such that a cell-membrane-impermeable compound that has a molecular weight of 300 or more is contained, in which the content of the cell-membrane-impermeable compound is 1.0 µmol/L or more and 100 µmol/L or less.

16. The preservative solution according to claim 15, wherein the preservative solution has the third configuration, and the cell-membrane-permeable compound is a polyhydric alcohol.

17. The preservative solution according to claim 15,
wherein the preservative solution has the third configuration, and the cell-membrane-permeable compound is ethylene glycol, propylene glycol, glycerin, dimethyl sulfoxide, acetamide, 1,3-propanediol or butylene glycol.

18. The preservative solution according to any one of claims 15 to 17, wherein the preservative solution has the fourth configuration, and the cell-membrane-impermeable compound is a compound having a molecular weight of 1000 or more.

19. The preservative solution according to any one of claims 15 to 18, wherein the preservative solution has the fourth configuration, and the cell-membrane-impermeable compound is polyvinylpyrrolidone, polyethylene glycol, polyvinyl alcohol, a polysaccharide, a derivative of a polysaccharide, a sugar alcohol or Ficoll.

20. The preservative solution according to any one of claims 15 to 19, wherein a formaldehyde donor compound is contained as a compound that is different from either of the cell-membrane-permeable compound and the cell-membrane-impermeable compound.

21. The preservative solution according to claim 20, wherein the formaldehyde donor compound is DMDM hydantoin or 1-hydroxymethyl-5,5-dimethylhydantoin.

22. The preservative solution according to any one of claims 15 to 21, wherein an osmotic pressure-controlling agent is contained as a compound that is different from either of the cell-membrane-permeable compound and the cell-membrane-impermeable compound, and the osmotic pressure-controlling agent is glucose or sodium chloride.

23. The preservative solution according to any one of claims 15 to 22, wherein a compound having a chelating activity is contained as a compound that is different from either of the cell-membrane-permeable compound and the cell-membrane-impermeable compound, and the compound having a chelating activity comprises EDTA.

24. The preservative solution according to any one of claims 15 to 23, wherein a buffering agent is contained as a compound that is different from either of the cell-membrane-permeable compound and the cell-membrane-impermeable compound, and the buffering agent comprises glycine.

25. The preservative solution according to any one of claims 15 to 24, wherein the preservative solution has the third configuration and the fourth configuration.

26. The preservative solution according to any one of claims 15 to 25, wherein the cell-containing solution is blood.
